# NEUE EUROPÄISCHE PATENTSCHRIFT

(11) **EP 1 722 763 B2**
(45) Veröffentlichungstag und Bekanntmachung des Hinweises auf die Entscheidung über den Einspruch: **01.08.2018**
(45) Hinweis auf die Patenterteilung: 29.04.2015
(21) Anmeldenummer: 05715565.7
(22) Anmeldetag: 25.02.2005
(51) Int. Cl.: A61K 9/70, A61L 15/44

(54) **PFLASTER MIT REDUZIERTER HAUTIRRITATION**
PLASTER THAT REDUCES SKIN IRRITATION
PANSEMENT ENTRAINANT UNE IRRITATION CUTANEE REDUITE

(30) Priorität: 26.02.2004 DE 102004009903
(43) Veröffentlichungstag der Anmeldung: 22.11.2006
(73) Patentinhaber: Grünenthal GmbH, 52078 Aachen (DE)
(72) Erfinder: KUGELMANN, Heinrich, 52068 Aachen (DE); BARTHOLOMÄUS, Johannes, 52080 Aachen (DE); SEDAGHAT KERDAR, Rasoul, 90455 Nürnberg (DE)
(74) Vertreter: Brosch, Oliver
(86) Internationale Anmeldenummer: PCT/EP2005/002032
(87) Internationale Veröffentlichungsnummer: WO 2005/082428

(56) Entgegenhaltungen:
- EP-A- 0 416 137
- EP-A- 0 607 434
- EP-A- 0 760 238
- WO-A-01/43717
- WO-A1-86/00532
- DE-A1- 3 333 444
- DE-A1- 4 241 874
- DE-A1- 10 056 010
- US-A- 4 573 996
- US-A- 4 690 683
- PATENT ABSTRACTS OF JAPAN Bd. 018, Nr. 651 (C-1285), 9. Dezember 1994 (1994-12-09) & JP 06 256183 A (SEKISUI CHEM CO LTD), 13. September 1994 (1994-09-13)
- PATENT ABSTRACTS OF JAPAN Bd. 1997, Nr. 04, 30. April 1997 (1997-04-30) & JP 08 319234 A (YUUTOKU YAKUHIN KOGYO KK), 3. Dezember 1996 (1996-12-03)

## Beschreibung

Die vorliegende Erfindung betrifft ein wirkstoffhaltiges Pflaster zur transdermalen Abgabe eines Analgetikums als pharmazeutischen Wirkstoff umfassend mindestens eine entfernbare Schutzfolie und eine Klebeschicht, dadurch gekennzeichnet, dass das Pflaster mindestens eine auf die Hautoberfläche übertragbare Hautirritationen wenigstens reduzierende Verbindung aufweist, die zwischen der Klebeschicht und der entfernbaren Schutzfolie an die Klebeschicht angrenzend gleichmäßig verteilt in Form eines Films oder einer Schicht oder punktuell verteilt über die Oberfläche der Klebeschicht und/oder in der Klebeschicht vorliegt, die separat von der wirkstoffhaltigen Schicht ist.

Es ist bekannt, dass der intensive Kontakt von Pflastern mit der Haut, insbesondere trockener oder vorgeschädigter Haut von Mensch oder Tier, zu Hautrötungen, Pusteln, Juckreiz oder anderen Zeichen der Hautirritation führen kann. Diese Hautreaktionen können bei bis zu 30% der Anwender von Pflastern in unterschiedlicher Stärke auftreten. Eine geringere Akzeptanz dieser Produkte ist die Folge, so dass es zu einem vorzeitigen Abbruch der Therapie kommen kann.

Es stellte sich daher die Aufgabe, ein wirkstoffhaltiges Pflaster zur Verfügung zu stellen, das diese Nachteile des Standes der Technik nicht aufweist, da es zu weniger Hautirritationen führt.

Diese Aufgabe wurde durch die Bereitstellung des erfindungsgemäßen wirkstoffhaltigen Pflaster zur transdermalen Abgabe eines Analgetikums als pharmazeutischen Wirkstoff umfassend mindestens eine entfernbare Schutzfolie und eine Klebeschicht, dadurch gekennzeichnet, dass das Pflaster mindestens eine auf die Hautoberfläche übertragbare Hautirritationen wenigstens reduzierende Verbindung aufweist, die zwischen der Klebeschicht und der entfernbaren Schutzfolie an die Klebeschicht angrenzend gleichmäßig verteilt in Form eines Films oder einer Schicht oder punktuell verteilt über die Oberfläche der Klebeschicht und/oder in der Klebeschicht vorliegt, die separat von der wirkstoffhaltigen Schicht ist, gelöst.

Das erfindungsgemäße Pflaster hat durch seinen Aufbau den Vorteil, dass die Hautirritation zumindest reduzierende Verbindung unmittelbar auf die Hautoberfläche beim Aufbringen des Pflasters übertragen wird und somit eine Hautirritation schon zu Anfang der Applikation verhindert wird, insbesondere dann, wenn die reduzierende Verbindung oder Substanz zwischen der Klebeschicht und der Schutzschicht vorliegt.

Die auf die Hautoberfläche zur Reduzierung oder Verhinderung von Hautirritationen übertragbare Verbindung ist vorzugsweise eine hautverträgliche Verbindung ausgewählt aus der Gruppe umfassend Paraffine, Silanole, Silikone, Silikon-Derivate, Mono-, Di- oder Polyalkohole, natürliche oder künstliche Lipide, natürliche oder künstliche Wachse, natürliche oder künstliche Fette, Fettsäuren und/ oder Fettalkohole, natürliche oder künstliche Öle, natürliche oder künstliche Polymere, Stärken, Proteine, Vitamine, Verbindungen mit entzündungsvorbeugenden oder antiphlogistischen Eigenschaften, Verbindungen zur Verhinderung des Wachstums von entzündungsverursachenden Mikroorganismen, Verbindungen mit anästhetisierenden Eigenschaften, Verbindungen wirksam als Radikalfänger, Enzyme, pflanzliche Extrakte, Konservierungsmittel und deren Mischungen aus wenigstens zwei Verbindungen einer Klasse oder wenigstens zweier unterschiedlicher Klassen von Verbindungen. Sofern Glyzerin als hautverträgliche, insbesondere hautglättende, Verbindung eingesetzt wird, wird diese nur in Kombination mit wenigstens einer weiteren Verbindung aus einer verschiedenen Klasse, vorzugsweise wenigstens einer Verbindung der nachstehend aufgeführten, bevorzugt zum Einsatz kommenden, Hautirritationen wenigstens reduzierenden Verbindungen, verwendet.

Der Begriff "Hautirritationen wenigstens reduzierende Verbindung" umfasst erfindungsgemäß daher auch Kombinationen der aufgeführten Verbindungen, die die angestrebte Wirkung zeigen.

Als Silikon-Derivate eignen sich vorzugsweise gegebenenfalls substituierte Polysiloxane, die gegebenenfalls mit Acrylatpolymeren gemischt sind.

Als Verbindungen mit entzündungsvorbeugenden oder antiphlogistischen Eigenschaften eignen sich vorzugsweise Allantoin, Dexpanthenol, Bisabolol, Chamazulen, Aescin, bas. Aluminiumacetat-tartrat, Zinkoxid, Gerbstoffe, Melatonin, Perubalsam, Bismutgallat, deren Derivate und/oder Salze, Corticoide, wie vorzugsweise Hydrocortison, Betamethason, Fluocinolonacetonid, Fluocinonid, Prednisolon, Methylprednisolon, Triamcinolon, Flumetason, Clobetasol, Flupredniden, Alclometason, Prednicarbat, Mometason, Fluticason, Halcinonid, Clocortolon, Diflucortolon, Desoximetason und/oder deren Derivate sowie Antihistaminica, wie vorzugsweise Diphenhydramin, Dimetinden, Isoprenalin, Clemastin, Bamipin, deren Derivate und/oder Salze.

Als Verbindungen zur Verhinderung des Wachstums von entzündungsverursachenden Mikroorganismen eignen sich vorzugsweise Benzalkoniumchlorid wie z. B. Benzethoniumchlorid, Methylhydroxybenzoat, Propylhydroxybenzoat, Chlorhexidin, Dequaliniumchlorid, Clioquinol, Sorbinsäure, deren Derivate und/oder Salze, Antiseptica, wie vorzugsweise Povidon-Jod, Jodoform, Thymol, Tyrothricin, Chlorocresol, Salicylsäure, Ethacridin oder Polidocanol, deren Derivate und/oder Salze, und Antiinfektiva, wie vorzugsweise Framycetin, Neomycin, Gentamicin, Nystatin, Erythromycin, Tetracyclin, Chlortetracyclin, Oxytetracyclin, Fusidinsäure, Metronidazol, Bacitracin-Zink, Miconazol, Amphotericin B, deren Derivate und/oder Salze.

Als Verbindungen mit anästhetisierenden Eigenschaften eignen sich vorzugsweise Benzocain, Lidocain, Tetracain, Prilocain, Mepivacain, deren Derivate und/oder Salze.

Als Vitamine eignen sich vorzugsweise Vitamin A Derivate, vorzugsweise Retinol-Acetat oder -Palmitat, Vitamin B Derivate, Vitamin C Derivate, wie z. B. das entsprechende Palmitat, Vitamin D Derivate, vorzugsweise Colecaliciferon oder Vitamin E Derivate, vorzugsweise α-Tocopherolacetat.

Als Enzyme eignen sich vorzugsweise Superoxid-Dismutase oder -Katalase.

Als pflanzliche Extrakte eignen sich vorzugsweise Extrakte aus Pflanzen wie z. B. Aloe Vera, Arnika, Basilikum, Schlehe (Lat.: Prunus spinosa), große Klette (Lat.: Arctium lappa), Ringelblume (Lat.: Calendula officinalis), Kamelie (Lat.: Camellia oleifera), Muskatellersalbei (Lat.: Salvia clarea), Kamille (Lat.: Matricaria chamomilla), Beinwell (Lat.: Symphytum officinale), Sonnenhut (Lat.: Echinacea angustifolia), Gurke (Lat.: Cucumis Sativus), Augentrost (Lat.: Euphrasia officinalis), Ginseng, grüner Tee, Lavendel, Kamille (Lat.: Chamomilla recutita und Matricaria chamomilla), Pfefferminze (Lat.: Mentha piperita), Beifuß, Muskat, Hafer (Lat.: Avena sativa), Sandelbaum, Florsafran (Lat.: Carthamus tinctorius), Soja, Teebaum (Lat.: Melaleuca alternifolia), Vetivergras (Lat.: Vetiveria zizanioides), Veilchen, Süßholz (Lat.: Glycyrrhiza glabra) und/oder Zaubernuss (Lat.: Hamamelis).

Von den genannten Verbindungen eignen sich vorzugsweise solche Verbindungen, die auch eine hautglättende Eigenschaft haben. So eignen sich auch als Hautirritationen wenigstens reduzierende Verbindung wenigstens eine hautglättende Verbindung bzw. Substanz aus der Gruppe umfassend Glycerin, Chitosan, Hydroxypropylmethylcellulose, Cetearyl Octanoat, Vitamin E, Kokosfett, Erdnußöl, Sojaöl und Butyrospermum Parkii (Shea Butter), wobei hydrophile Verbindungen, wie Glyzerin, nur in Kombination mit einer der vorstehend genannten, Hautirritationen reduzierenden Verbindungen, eingesetzt werden.
Besonders bevorzugt ist wenigstens eine hautverträgliche Verbindung ausgewählt aus der Gruppe umfassend polymere Verbindungen, vorzugsweise fluorierte Polyether, besonders bevorzugt Polyperfluormethylisopropylether, oder Silikon-Derivate; Verbindungen mit entzündungsvorbeugenden oder antiphlogistischen Eigenschaften, vorzugsweise Corticoide oder Antihistaminica; Verbindungen zur Verhinderung des Wachstums von entzündungsverursachenden Mikroorganismen, vorzugsweise Antiseptica oder Antiinfektiva, und Verbindungen wirksam als Radikalfänger, vorzugsweise N-Acylethanolamin, geeignet.

Das erfindungsgemäße, wirkstoffhaltige Pflaster kann nach dem Reservoir- oder Matrix- System aufgebaut sein (Bauer K. H., Frömming K.-H., Führer C., Pharmazeutische Technologie, Seiten 381-383; Müller R. H., Hildebrand G. E., Pharmazeutische Technologie: Moderne Arzneiformen, Kapitel 8).

Gemäß dem Matrix-System kann das wirkstoffhaltige Pflaster vorzugsweise eine Trägerschicht, eine wirkstoffhaltige Schicht, eine Klebeschicht und eine entfernbare Schutzfolie aufweisen.

Die Klebeschicht und die wirkstoffhaltige Schicht liegen als getrennte Schichten vor, wobei die Klebeschicht flächendeckend, partiell oder ringförmig auf der wirkstoffhaltigen Schicht aufgebracht sein kann.

Wenn das erfindungsgemäße Pflaster nach dem Reservoir-System aufgebaut ist, kann die Klebeschicht flächendeckend auf oder ringförmig um die Reservoir-Membran des Reservoir-Systems aufgebracht sein.

Wenigstens eine auf die Hautoberfläche übertragbare, Hautirritationen wenigstens reduzierende, hautverträgliche Verbindung bzw. Substanz liegt als Komponente in der Klebeschicht und/oder an der Klebeschicht zwischen Klebeschicht und Schutzfolie des erfindungsgemäßen Pflasters vor.

Wenn die Verbindung in der Klebeschicht vorliegt, kann sie darin gelöst und/oder dispergiert sein.

Es ist aber auch möglich, dass die Verbindung bzw. Substanz auch gegebenenfalls nur in einem Teilsektor der Klebeschicht so vorliegt, dass dieser Teilsektor als Reservoir nur für die hautverträgliche Verbindung gestaltet ist.

Vorzugsweise befindet sich die auf die Hautoberfläche übertragbare, Hautirritationen wenigstens reduzierende Verbindung bzw. Substanz an der Klebeschicht angrenzend zwischen der Klebeschicht und der Schutzfolie, wobei beim Abziehen der Schutzfolie die Verbindung zunächst auf der Klebeschicht verbleibt und bei der Applikation sofort auf die Hautoberfläche übertragen wird. Vorzugsweise ist die Anordnung der hautverträglichen Verbindung auf der Klebeschicht entsprechend der Anordnung der Klebeschicht in dem erfindungsgemäßen Pflaster.

Wenn die auf die Hautoberfläche übertragbare, Hautirritationen wenigstens reduzierende, hautverträgliche Verbindung an der Klebeschicht angrenzend zwischen dieser und der Schutzfolie vorliegt, kann sie entsprechend der Anordnung der Klebeschicht gleichmäßig verteilt, vorzugsweise flächendeckend in Form eines Films oder eine Schicht, oder punktuell, beispielsweise in multipartikulärer Form, verteilt über die Oberfläche der Klebeschicht aufgebracht sein.

Wenn die auf die Hautoberfläche übertragbare, Hautirritationen wenigstens reduzierende, hautverträgliche Verbindung in multipartikulärer Form vorliegt, kann sie vorzugsweise in Mikro- bzw. Nano-Kapseln, Mikro- bzw. Nano-Partikeln oder Liposomen vorliegen.

Wie bereits erwähnt, kann die auf die Hautoberfläche übertragbare, Hautirritationen wenigstens reduzierende Verbindung bzw. Substanz vorzugsweise als eine Schicht mit vorzugsweise einer Dicke < 5 µm, besonders bevorzugt von 0,5-2 µm zwischen der entfernbaren Schutzfolie und Klebeschicht vorliegen.

Für den Fachmann ist es selbstverständlich, dass die auf die Hautoberfläche übertragbare, Hautirritationen wenigstens reduzierende Verbindung, sei diese Verbindung in oder an der Klebeschicht, nur in einer solchen Menge vorliegt, dass die Klebewirkung der Klebeschicht nicht oder höchstens nur geringfügig beeinträchtigt wird, aber die angestrebte Wirkung eintritt.

Als Klebstoffe zur Herstellung der Klebeschicht des erfindungsgemäßen Pflasters werden vorzugsweise druckempfindliche Klebemittel ("pressure-sensitive adhesives") eingesetzt. Beispielsweise eignen sich dafür Polymere wie Polyacrylate, Polyvinylether, Polyisobutylene (PIB), Styrol/Isopren- oder Butadien-/Styrol Copolymere oder Polyisopren Kautschuke. Weiterhin eignen sich Silikon-Klebstoffe, wie z. B. gegebenenfalls vernetzte Polydimethylsiloxane. Ferner sind Harze wie z. B. Polyester von Glycinen, Glycerin oder Pentaerythrol, oder Kohlenwasserstoff-Harze wie Polyterpene geeignet. Klebstoffe auf Acrylatbasis werden durch Polymerisation von Acrylaten, Methacrylaten, Alkylacrylaten und/oder Alkylmethacrylaten, mit gegebenenfalls weiteren α, β ungesättigten Monomeren wie Acrylamid, Dimethylacrylamid, Dimethylaminoethylacrylat,Hydroxyethylacrylat, Hydroxypropylacrylat, Methoxyethylacrylat, Methoxyethylmethacrylat, Acrylnitril und/oder Vinylacetat, hergestellt.

Die Klebeschicht kann zusätzlich Hilfsstoffe wie Weichmacher, z. B. Phthalate wie Dibutylphthalate, Mineralöle, Ester von Zitronensäure, oder Ester von Glycerin, Hautdurchdringungsverstärker, Füllstoffe (wie Zinkoxid oder Silika), Vernetzer, Konservierungsmittel und/oder lipophile Lösungsmittel enthalten.

Die Trägerschicht bzw. Deckschicht des erfindungsgemäßen Pflasters ist vorzugsweise für die in der wirkstoffhaltigen Schicht und in der Klebeschicht enthaltenen Verbindungen undurchlässig und inert und kann aus Polymeren wie Polyester, z. B. Polyethylenphthalat, Polyolefinen, wie Polyethylenen, Polypropylenen oder Polybutylenen, Polycarbonaten, Polyethylenoxiden, Polyterephthalaten wie Polyethylenterephthalaten, Polyurethanen, Polystyrolen, Polyamiden, Polyimiden, Polyvinylacetaten, Polyvinylchloriden und/oder Polyvinylidenchloriden, Copolymeren wie Acrylonitril/Butadien/Styrol Copolymeren enthaltend Papierfasern, Textilfasern und/oder deren Mischungen bestehen, die bei Bedarf metallisiert oder pigmentiert sein können. Die Trägerschicht kann auch aus einer Kombination aus einer Metallfolie und einer Polymerschicht bestehen.

Das erfindungsgemäße Pflaster eignet sich für die transdermale Verabreichung eines jeden systemisch, d. h. transdermal wirksamen, pharmazeutischen Analgetikums. Vorzugsweise eignet sich das erfindungsgemäße Pflaster zur transdermalen (systemischen) Abgabe von wenigstens einem Analgetikum als pharmazeutischen Wirkstoff.

Besonders bevorzugt eignet es sich zur transdermalen Applikation von Opioiden, wie z. B. Buprenorphin, Fentanyl oder Morphin.

Die jeweilige Dosierung des transdermal zu verabreichenden Analgetikums ist dem Fachmann bekannt und wird u. a. von der Applikationsdauer beeinflusst.

Die wirkstoffhaltige Matrix-Schicht des erfindungsgemäßen Pflasters kann neben wenigstens einem Analgetikum als pharmazeutischen Wirkstoff auch matrixbildende Polymere, Weichmacher, Permeationsförderer, lipophile Lösungsvermittler, Vernetzer, Konservierungsmittel, Emulgatoren, Konservierungsmittel, Verdickungsmittel enthalten.

Als matrixbildende Polymere können üblicherweise filmbildende Polymere wie z. B. Hydroxypropylcellulose, Carboxymethylcellulose, Polyethylene, chlorierte Polyethylene, Polypropylene, Polyuretane, Polycarbonate, Polyacrylsäureester, Polyacrylate, Polymethacrylate, Polyvinylalkohole, Polyvinylchloride, Polyvinylidenchloride, Polyvinylpyrrolidone, Polyethylentherephthalate, Polytetrafluoroethylene, Ethylen/Propylen Copolymere, Ethylen/Ethylacrylat Copolymere, Ethylen/Vinylacetat Copolymere, Ethylen/Vinylalkohol Copolymere, Ethylen/Vinyloxyethanol Copolymere, Vinylchlorid/Vinylacetat Copolymere, Vinylpyrrolidon/Ethylen/Vinylacetat Copolymere, Kautschuke, gummi-artige synthetische Homo-, Co- oder Blockpolymere, Silikone, Silikon-Derivate wie Polysiloxan/Polymethacrylat Copolymere, Cellulose-Derivate wie Ethylcellulose oder Celluloseether und/oder deren Mischungen eingesetzt werden.

Als lipophile Lösungsvermittler können N-methyl-2-pyrrolidon, Laurylpyrrolidon, Triacetin, Diethylenglykol-monoethylether, Derivate von Fettsäuren oder Fettalkoholen verwendet werden.

Wenn das erfindungsgemäße, wirkstoffhaltige Pflaster nach dem Reservoir-System aufgebaut ist, kann die Reservoir-Membran aus inerten Polymeren, wie z. B. Polyethylenen, Polypropylenen, Polyvinylacetaten, Polyamiden, Ethylen/Vinylacetat Copolymeren und/oder Silikonen bestehen. Durch die Reservoir-Membran kann aus dem Reservoir eine kontrollierte Freisetzung des Analgetikums erzielt werden.

Das wirkstoffhaltige Reservoir kann auch ein Lösungsmittel, wie z. B. Wasser, Ethanol, 1-Propanol, Isopropanol, einen niedermolekularen, mehrwertigen Alkohol, beispielsweise Propylenglycol oder Glycerin, oder einen Ester, wie Isopropylmyristat, oder deren Mischungen in üblichen Mengen enthalten.

Als Konservierungsmittel für wirkstoffhaltige Matrix bzw. das wirkstoffhaltige Reservoir können Antioxidantien, wie Vitamin E, Butylhydroxytoluol, Butylhydroxyanisol, Ascorbinsäure, Ascorbylpalmitat, und/oder Chelatbildner, wie Dinatriumethylendiamintetraessigsäure, Kalium- oder Natriumcitrat in bekannten, üblichen Mengen verwendet werden.

Die wirkstoffhaltige Matrix bzw. das wirkstoffhaltige Reservoir kann auch bekannte, übliche Permeationsförderer und/oder viskositätserhöhende Mittel, wie z. B. Cellulose-Derivate oder natürliche oder synthetische Kautschuke, enthalten. Vorzugsweise liegt das pharmazeutische Analgetikum in dem erfindungsgemäßen Pflaster in einer Matrix-Schicht vor.

Die Wirkstofffreisetzung aus dem erfindungsgemäßen Pflaster ist vorzugsweise kontrolliert.

Das erfindungsgemäße Pflaster kann in einer Schicht oder mehreren Schichten auch wenigstens einen Weichmacher in üblichen Mengen ausgewählt aus der Gruppe umfassend langkettige Alkohole wie Dodecanol, Undecanol, Octanol, Ester von Carbonsäuren mit polyethoxylierten Alkoholen sein sollte, Diester von aliphatischen Dicarbonsäuren wie Adipinsäure, und mittelkettige Triglyceride von Caprylsäure und/oder Caprinsäure, Kokosfett, mehrwertige Alkohole wie 1,2 - Propandiol, Ester von mehrwertigen Alkoholen wie Glycerin mit Lävulinsäure oder Caprylsäure, und veretherte mehrwertige Alkohole enthalten.

Die Schutzfolie kann aus Polyethylen, Polyester, Polyethylenterephthalat, Polypropylen, Polysiloxan, Polyvinylchlorid oder Polyurethan und gegebenenfalls aus behandelten Papierfasern, wie z. B. Zellophan, bestehen und gegebenenfalls eine Silikon-, Fluorsilikon- oder Fluorcarbonbeschichtung aufweisen.

Die Herstellung des erfindungsgemäßen Pflasters erfolgt nach den bekannten Herstellungsverfahren für Pflastern mit Verfahrensschritten wie Laminieren, Stanzen, Delaminieren, Abwickeln, Schneiden, Wiederaufwickeln, Montieren oder Dosieren (vgl. Verpackungs-Rundschau 4/2002, 83-84).

Das erfindungsgemäße Pflaster kann bei Mensch und Tier appliziert werden, wobei Hautirritationen, hervorgerufen durch eine Pflasterapplikation, weitgehend vermieden werden.

### Beispiele

### Beispiel 1

Ein Matrixpflaster, das 0,5 Gew.% Capsaicin als Wirkstoff enthält und eine Klebeschicht auf Basis eines Polyacrylats über der wirkstoffhaltigen Schicht und gegebenenfalls über der Klebeschicht zwischen Klebeschicht und Schutzfolie eine Schicht aus Polyperfluormethylisopropylether mit einer Dicke von ungefähr 1 µm aufweist, wurde entweder mit der die Hautirritationen verhindernden Schicht oder ohne diese Schicht getestet.

Dabei testeten 5 Personen jeweils für 3 Tage ein Pflaster mit bzw. ohne ein Hautirritationen verhinderndes Mittel, indem sie auf einer nicht behaarten Fläche des Unterarms die entsprechenden Pflaster mit einer Größe von 2 x 2 cm nach Entfernung der Schutzfolie aufklebten.
Nach 3 Tagen wurden die Pflaster entfernt und die Hautirritationen unmittelbar danach und nach weiteren 24 Stunden bewertet.

| Pflaster ohne Polyperfluormethylisopropyletherschicht | | | Pflaster mit | |
|---|---|---|---|---|
| Person | nach 3 Tagen | nach 4 Tagen | nach 3 Tagen | nach 4 Tagen |
| 1 | +++ | +++ | (+) | - |
| 2 | ++ | +++ | - | - |
| 3 | +++ | ++ | - | - |
| 4 | ++++ | +++ | (+) | - |
| 5 | ++ | +++ | - | - |

| | | | | |
|---|---|---|---|---|
| - = keine Hautirritationen (+) = kaum sichtbare Hautirritationen + = schwache Hautirritationen ++ = einzelne starke Hautirritationen +++ = starke Hautirritationen ++++ = starke Hautirritationen mit Pusteln | | | | |

Das Testergebnis bestätigt, dass Pflaster mit einer Schicht einer Hautirritationen verhindernden Verbindung diese Irritationen tatsächlich weitgehend verhindern.

## Patentansprüche

1. Ein wirkstoffhaltiges Pflaster zur transdermalen Abgabe eines Analgetikums als pharmazeutischer Wirkstoff umfassend mindestens eine entfernbare Schutzfolie und eine Klebeschicht, **dadurch gekennzeichnet, dass** das Pflaster mindestens eine auf die Hautoberfläche übertragbare Hautirritationen wenigstens reduzierende Verbindung aufweist, die zwischen der Klebeschicht und der entfernbaren Schutzfolie an die Klebeschicht angrenzend gleichmäßig verteilt in Form eines Films oder einer Schicht oder punktuell verteilt über die Oberfläche der Klebeschicht und/oder
in der Klebeschicht vorliegt, die separat von der wirkstoffhaltigen Schicht ist.

2. Pflaster nach Anspruch 1, **dadurch gekennzeichnet, dass** es eine wirkstoffhaltige Schicht, eine separate Klebeschicht und eine Trägerschicht umfasst.

3. Pflaster nach Anspruch 1 oder 2, **dadurch gekennzeichnet, dass** die Hautirritationen wenigstens reduzierende Verbindung als eine Komponente in der Klebeschicht vorliegt.

4. Pflaster gemäß Anspruch 3, **dadurch gekennzeichnet, dass** die Hautirritationen wenigstens reduzierende Verbindung in der Klebeschicht in wenigstens einem Teilsektor vorliegt.

5. Pflaster gemäß Anspruch 4, **dadurch gekennzeichnet, dass** der Teilsektor als Reservoir nur die Hautirritationen wenigstens reduzierende Verbindung aufweist.

6. Pflaster gemäß einem der Ansprüche 1 bis 5, **dadurch gekennzeichnet, dass** die Hautirritationen reduzierende Verbindung in einer Menge vorliegt, die die Klebewirkung der Klebeschicht nicht oder nur gering beeinträchtigt.

7. Pflaster gemäß einem der Ansprüche 1 bis 6, **dadurch gekennzeichnet, dass** als Hautirritationen wenigstens reduzierende Verbindung wenigstens eine Verbindung oder eine Substanz ausgewählt aus der Gruppe umfassend
Paraffine, Silanole, Silikone, Silikon-Derivate, hautverträgliche Mono- Di- oder Polyalkohole, natürliche oder künstliche Lipide, natürliche oder künstliche Wachse, natürliche oder künstliche Fette, Fettsäuren, Fettalkohole, natürliche oder künstliche Öle, natürliche oder künstliche Polymere, Stärken, Proteine, Vitamine, Verbindungen mit entzündungsvorbeugenden oder antiphlogistischen Eigenschaften, Verbindungen zur Verhinderung des Wachstums von entzündungsverursachenden Mikroorganismen, Verbindungen mit anästhetisierenden Eigenschaften, Verbindungen wirksam als Radikalfänger, Enzyme, pflanzliche Extrakte, Konservierungsmittel und deren Mischungen aus wenigstens zwei Verbindungen einer Klasse oder wenigstens zweier unterschiedlicher Klassen vorliegt.

8. Pflaster gemäß einem der Ansprüche 1 bis 7, **dadurch gekennzeichnet, dass** die Hautirritationen wenigstens reduzierende Verbindung eine hautglättende Verbindung ist, wobei Glycerin nur in Kombination mit wenigstens einer weiteren Verbindung aus einer unterschiedlichen Klasse von Hautirritationen wenigstens reduzierenden Verbindungen eingesetzt wird.

9. Pflaster gemäß Anspruch 7 oder 8, **dadurch gekennzeichnet, dass** die Hautirritationen wenigstens reduzierende Verbindung ein fluorierter Polyether, vorzugsweise Polyperfluormethylisopropylether, ein Silikonderivat, ein Cortidoid, ein Antihistamin, ein Antiseptikum, ein Antiinfektivum, eine Radikalfängerverbindung, vorzugsweise N-Acylethanolamin, oder eine Mischung aus wenigstens 2 dieser Verbindungen ist.

10. Pflaster gemäß einem der Ansprüche 1-9, **dadurch gekennzeichnet, dass** das Analgetikum wenigstens ein Opioid ist.

11. Pflaster nach Anspruch 10, **dadurch gekennzeichnet, dass** das Opioid Buprenorphin ist.

## Claims

1. An active substance plaster for the transdermal release of an analgesic as pharmaceutical active substance comprising at least one removable protective film and an adhesive layer, **characterised in that** the plaster comprises at least one compound which at least reduces skin irritations that are transferable to the surface of the skin and which is distributed evenly over the surface of the adhesive layer, between the adhesive layer and the removable protective film and adjoining the adhesive layer, in the form of a film or a layer or in dot form and/or is present in the adhesive layer, which is separate from the layer containing the active substance.

2. Plaster according to claim 1, **characterised in that** it comprises a layer containing the active substance, a separate adhesive layer and a substrate layer.

3. Plaster according to claim 1 or 2, **characterised in that** the compound that at least reduces skin irritations is present as a component in the adhesive layer.

4. Plaster according to claim 3, **characterised in that** the compound that at least reduces skin irritations is present in the adhesive layer in at least one partial sector.

5. Plaster according to claim 4, **characterised in that** the partial sector as a reservoir contains only the compound that at least reduces skin irritations.

6. Plaster according to any one of claims 1 to 5, **characterised in that** the skin irritation-reducing compound is present in an amount that does not impair, or only slightly impairs, the adhesive action of the adhesive layer.

7. Plaster according to any one of claims 1 to 6, **characterised in that** at least one compound or substance selected from the group consisting of
paraffins, silanols, silicones, silicone derivatives, skin-friendly monohydric, dihydric or polyhydric alcohols, natural or synthetic lipids, natural or synthetic waxes, natural or synthetic fats, fatty acids, fatty alcohols, natural or synthetic oils, natural or synthetic polymers, starches, proteins, vitamins, compounds with antiinflammatory or antiphlogistic characteristics, compounds for preventing the growth of phlogogenic microorganisms, compounds with anaesthetising characteristics, compounds that are effective as free-radical scavengers, enzymes, plant extracts, preservatives and mixtures thereof consisting of at least two compounds of one class or of at least two different classes
is present as compound that at least reduces skin irritations.

8. Plaster according to any one of claims 1 to 7, **characterised in that** the compound that at least reduces skin irritations is a skin-smoothing compound, wherein glycerin is used only in combination with at least one further compound from a different class of compounds that at least reduce skin irritations.

9. Plaster according to claim 7 or 8, **characterised in that** said compound that at least reduces skin irritations is a fluorinated polyether, preferably polyperfluoro-methylisopropyl ether, a silicone derivative, a corticoid, an antihistamine, an antiseptic, an anti-infective, a free-radical scavenger compound, preferably N-acyl ethanolamine, or a mixture of at least two of these compounds.

10. Plaster according to any one of claims 1 to 9, **characterised in that** the analgesic is at least one opioid.

11. Plaster according to claim 10, **characterised in that** the opioid is buprenorphine.

## Revendications

1. Pansement contenant des substances actives pour l'administration transdermique d'un analgésique en tant que substance active pharmaceutique comprenant au moins une feuille de protection amovible et une couche adhésive, **caractérisé en ce que** le pansement présente au moins un composé réduisant au moins les irritations de la peau transférables à la surface de la peau, qui se trouve entre la couche adhésive et la feuille de protection amovible, en jouxtant la couche adhésive, sous forme régulièrement répartie sous forme d'un film ou d'une couche ou réparti de manière ponctuelle sur la surface de la couche adhésive et/ou dans la couche adhésive, qui est séparée de la couche contenant une substance active.

2. Pansement selon la revendication 1, **caractérisé en ce qu'**il contient une couche contenant une substance active, une couche adhésive séparée et une couche support.

3. Pansement selon la revendication 1 ou 2, **caractérisé en ce que** le composé réduisant au moins les irritations de la peau se trouve sous forme d'un composant dans la couche adhésive.

4. Pansement selon la revendication 3, **caractérisé en ce que** le composé réduisant au moins les irritations de la peau se trouve dans la couche adhésive dans au moins un secteur partiel.

5. Pansement selon la revendication 4, **caractérisé en ce que** le secteur partiel présente, comme réservoir, uniquement le composé réduisant au moins les irritations de la peau.

6. Pansement selon l'une quelconque des revendications 1 à 5, **caractérisé en ce que** le composé réduisant les irritations de la peau se trouve en une quantité qui ne compromet pas ou alors seulement faiblement l'effet adhésif de la couche adhésive.

7. Pansement selon l'une quelconque des revendications 1 à 6, **caractérisé en ce qu'**il s'agit pour le composé réduisant au moins les irritations de la peau au moins d'un composé ou d'une substance choisi(e) dans le groupe comprenant
les paraffines, les silanols, les silicones, les dérivés de silicone, les monoalcools, dialcools ou polyalcools tolérés par la peau, les lipides naturels ou synthétiques, les cires naturelles ou synthétiques, les graisses naturelles ou synthétiques, les acides gras, les alcools gras, les huiles naturelles ou synthétiques, les polymères naturels ou synthétiques, les amidons, les protéines, les vitamines, les composés présentant des propriétés anti-inflammatoires ou antiphlogistiques, les composés pour empêcher la croissance de micro-organismes provoquant une inflammation, les composés présentant des propriétés anesthésiantes, les composés actifs comme pièges de radicaux, les enzymes, les extraits végétaux, les conservateurs et les mélanges d'au moins deux composés d'une classe ou d'au moins deux classes différentes.

8. Pansement selon l'une quelconque des revendications 1 à 7, **caractérisé en ce que** le composé réduisant au moins les irritations de la peau est un composé lissant la peau, le glycérol n'étant utilisé qu'en combinaison avec au moins un autre composé d'une classe différente de composés réduisant au moins les irritations de la peau.

9. Pansement selon la revendication 7 ou 8, **caractérisé en ce que** le composé réduisant au moins les irritations de la peau est un polyéther fluoré, de préférence le polyperfluorométhylisopropyléther, un dérivé de silicone, un corticoïde, un antihistaminique, un antiseptique, un anti-infectieux, un composé piège de radicaux, de préférence la N-acyléthanolamine ou un mélange d'au moins 2 de ces composés.

10. Pansement selon l'une quelconque des revendications 1 à 9, **caractérisé en ce que** l'analgésique est au moins un opiacé.

11. Pansement selon la revendication 10, **caractérisé en ce que** l'opiacé est la buprénorphine.
